# EUROPEAN PATENT SPECIFICATION

(11) **EP 3 923 928 B1**
(45) Date of publication and mention of the grant of the patent: **08.10.2025**
(21) Application number: 20755431.2
(22) Date of filing: 18.02.2020
(51) Int. Cl.: A61K 31/337, A61K 9/10, C07K 16/22, A61K 9/19, A61L 27/52, A61L 27/56

(54) **METHODS FOR CONVERTING COLLOIDAL SYSTEMS TO RESUSPENDABLE/REDISPERSIBLE POWDERS THAT PRESERVE THE ORIGINAL PROPERTIES OF THE COLLOIDS**
VERFAHREN ZUR UMWANDLUNG VON KOLLOIDALEN SYSTEMEN IN RESUSPENDIERBARE/REDISPERGIERBARE PULVER, DIE DIE URSPRÜNGLICHEN EIGENSCHAFTEN DER KOLLOIDE BEWAHREN
PROCÉDÉS DE CONVERSION DE SYSTÈMES COLLOÏDAUX EN POUDRES POUVANT ÊTRE REMISES EN SUSPENSION/REDISPERSIBLES QUI PRÉSERVENT LES PROPRIÉTÉS ORIGINALES DES COLLOÏDES

(30) Priority: 15.02.2019 US 201962806582 P
(43) Date of publication of application: 22.12.2021
(73) Proprietor: THE REGENTS OF THE UNIVERSITY OF CALIFORNIA, Oakland, CA 94607-5200 (US)
(72) Inventor: SHEIKHI, Amir, State College, Pennsylvania 16801 (US); DICARLO, Dino, Los Angeles, California 90095 (US); KHADEMHOSSEINI, Alireza, Los Angeles, California 90049 (US)
(74) Representative: Murgitroyd & Company
(86) International application number: PCT/US2020/018622
(87) International publication number: WO 2020/168338

(56) References cited:
- WO-A1-2017/158482
- WO-A1-2019/039483
- US-A1- 2008 286 280
- US-B2- 9 822 243
- ZHANG HAIFEI, WANG DONG, BUTLER RACHEL, CAMPBELL NEIL L., LONG JAMES, TAN BIEN, DUNCALF DAVID J., FOSTER ALISON J., HOPKINSON ANDR: "Formation and enhanced biocidal activity of water-dispersable organic nanoparticles", NATURE NANOTECHNOLOGY, vol. 3, 6 July 2008 (2008-07-06), pages 506 - 511, XP002726349
- SHEIKHI AMIR, LISA DONATELLA DI, RUTTE JOSEPH DE, AKOUISSI OUTMAN, DI CARLO DINO, KHADEMHOSSEINI ALI: "Microengineered Emulsion-to-Powder Technology for the High-Fidelity Preservation of Molecular, Colloidal, and Bulk Properties of Hydrogel Suspensions", ACS APPLIED POLYMER MATERIALS, vol. 1, 5 July 2019 (2019-07-05), pages 1935 - 1941, XP055732753

## Description

### TECHNICAL FIELD

The invention relates to methods useful for converting colloidal systems to resuspendable/redispersible powders.

### BACKGROUND OF THE INVENTION

Emulsion and colloidal dispersions/suspensions have gained a tremendous importance in a myriad of applications and industries, including biomedicine (1,2), pharmaceutics (3,4), oil and gas processing (5), food formulation (6,7), and cosmetics (8,9). Emulsions are typically a mixture of two immiscible liquids often interfacially stabilized by a surfactant (10). Common emulsions are oil-in-water, wherein oil is the disperse phase distributed in a continuous aqueous phase, and water-in-oil emulsions, which comprise a dispersed aqueous phase in a continuous oil phase. Water-in-oil emulsions have forged the foundation of droplet-based microfluidics (11-14), wherein a miniature volume of an aqueous phase is surrounded by a continuous oil flow, forming uniform-sized droplets, which have been used for drug/gene/RNA delivery platforms (15), antibacterial agents (16), material fabrication and synthesis (17,18), chemical catalysis (19,20), biochemical analyses (21), cell analyses and sorting (22-24), and optical and plasmonic devices (25,26).

Colloids, e.g., solid-liquid suspensions are often post-processed to separate the physically- and/or chemically stabilized dispersed (target) phase from the impurities (e.g., oil and surfactant), which is used directly or following the isolation of encapsulated species. The separation process typically involves the addition of an external agent to break the emulsion followed by several steps of washing to yield the target phase in an aqueous medium. One of the notable examples for such process is the fabrication of dual-reactive hydrogel microspheres that can initially be crosslinked to form a colloidal suspension/dispersion and permit the removal of continuous phase, followed by secondary reactions/binding to form a three-dimensional microporous hydrogel construct (27-30). This emerging class of hydrogels have overcome some of the most persistent challenges of conventional, bulk hydrogels, such as porosity-stiffness correlation, injectability, and micrometer scale porosity.

Despite the widespread applications of water-in-oil emulsions, particularly microfluidic-enabled systems, challenges associated with the sterilization of aqueous-based products, the short lifetime of active substance in aqueous media, bacterial and viral contamination in aqueous conditions, poor long-term stability, and the necessity for having in-house microfluidic manufacturing capabilities have limited the use of microfluidic-enabled materials (31,32). Separating the dispersed phase of emulsions, particularly when the particle size is small, has been extremely cumbersome (33). Converting a stabilized dispersed phase to a solid phase may overcome all these challenges; however, current efforts for drying emulsions, colloidal dispersions and suspensions mainly rely on solvent evaporation and spray-drying (34), freeze-drying (35-38), electrospinning (39), and electrospraying (40), none of which have been able to decently preserve the physical and/or chemical properties of the dispersed phase. Importantly, cryoprotectants, e.g., polyethylene glycol (PEG), are highly prone to vitrification and have only been able to partially protect the dispersed phases in an emulsion during the freeze-drying when the freezing rate is low and/or the concentration of the additive is very high, e.g., 40 wt% (41). To this end, hydrogel particles are among the most challenging class of materials to be converted to a dry state that can recover all physicochemical properties when rehydrated.

There is a need in this field of technology for new methods and materials useful for converting colloidal systems to resuspendable/redispersible powders that retain material properties and shape following resuspension.

WO 2017/158482 A1 discloses emulsion compositions comprising a continuous oil phase, a discontinuous aqueous phase and a plurality of microparticles. The composition may comprise a pharmaceutical active ingredient located in the oil phase, the particulate phase or the aqueous phase. Zhang Haifei et al., Nature Nanotechnology, vol. 3, 6 July 2008, pages 506-511, discloses the formation of organic nanodispersions by freeze-drying emulsions containing water-insoluble compounds dissolved in a volatile oil phase. Watersoluble materials present in the aqueous phase form a porous solid support that dissolves rapidly on addition of water to disperse the nanoparticles. US 2008/286280 A1 discloses a process for the production of lyophilized pharmaceutical preparations of monoclonal or polyclonal antibodies which contain a sugar or an amino sugar, an amino acid and a surfactant as stabilizers. US 9 822 243 B2 discloses a process for the preparation of freeze-dried compositions wherein the compositions comprise at least one polymer based on polyacrylic acids and salts thereof and at least one natural polymer. The process comprises preparing an aqueous suspension or a solution of the at least one natural polymer, adjusting the pH of the aqueous suspension or solution to a pH of from pH 3.0 to pH 6.5, preparing an aqueous suspension or a solution of the polymer based on polyacrylic acids and salts thereof and adjusting the pH of the solution to a pH of between pH 3.0 and pH 6.5 with alkalis, combining the suspensions or solutions, pouring into a suitable mould or on to a suitable surface, freezing and freeze drying to form the freeze-dried composition.

### SUMMARY OF THE INVENTION

The invention disclosed herein provides methods useful to convert various dispersions of lyophilizable agents into powders that can readily be re-dispersed in liquid media to restore the original properties of these agents, such as shape, size, functionality, stiffness, biological cues, and the like. These methods involve stabilizing lyophilizable agents that are disposed in a dispersed aqueous phase within a continuous oil phase, followed by freezing this multiphase system, and then removing oils and water by drying to yield a micro-engineered powder. When the powders generated by this methodology are re-suspended, the resuspended agents unexpectedly exhibit the desirable/original material properties of the agents that are observed prior to lyophilization.

Disclosed herein are methods of making a lyophilized composition as claimed in claim 1. These methods comprise combining together an oil, a surfactant, a lyophilizable agent and an aqueous solution so as to form a multiphase system comprising the lyophilizable agent dispersed within an aqueous phase (e.g. dispersions comprising the lyophilizable agent within droplets of an aqueous solution) that is stabilized within a continuous oil/surfactant phase. These methods further include freezing this multiphase system; and then removing water, oil and surfactant from the multiphase system using a drying process so that the lyophilized composition is made.

The invention also includes methods of making a resuspended lyophilized composition as claimed in claim 12. These methods comprise combining a lyophilized composition with an aqueous resuspension solution such that a resuspended lyophilized composition is made. In these methods, the lyophilized composition that is resuspended is made by the method of any one of claims 1 to 9 comprising combining together an oil, a surfactant, a lyophilizable agent and an aqueous solution so as to form a multiphase system comprising the lyophilizable agent dispersed within an aqueous phase that is stabilized within a continuous oil/surfactant phase; freezing this multiphase system; and then removing water, oil and surfactant from the multiphase system using a drying process so that the lyophilized composition is made. In illustrative working embodiments of these methods that are disclosed herein, the lyophilizable agent comprises hydrogel particles and the aqueous resuspension solution comprises a crosslinking agent. In such methods where the lyophilizable agent comprises hydrogel particles, the method can further entail crosslinking the hydrogel particles so as to form a microporous hydrogel scaffold having properties that are equivalent to microporous hydrogel scaffolds made from control hydrogel particles that have not been lyophilized. Typically in these embodiments of the invention, the microporous hydrogel scaffolds exhibit a compression modulus that is at least 80% the compression modulus observed in a control microporous hydrogels formed from identical hydrogel particles that have not been lyophilized.

The illustrative working embodiments of the invention that are discussed below provide novel procedures to prepare lyophilized composition powders that can generate annealable uniform-sized hydrogel particles following their dispersal in a liquid media. These methods are based on stabilizing microfluidic-made polymeric particles (in an aqueous phase) in an oil/surfactant (e.g. oil-surfactant such as Novec 7500^{™} and 0.5 wt% PicoSurf), followed by deep freezing the aqueous phase (e.g. at -80 °C). Different freezing techniques, such as freezing at 0 °C, or at -80 °C followed by -196 °C, may be used in embodiments of the invention. While 0.5 wt% PicoSurf is used in the working embodiments of the invention, other types of oils and surfactants may also be used to stabilize the initial hydrogel particle suspensions and permit the sublimation and/or evaporation of the oil phase. The frozen samples are then lyophilized under vacuum (e.g., 0.006 mbar) to remove both water and oil phases using a freeze-dryer to yield a powder.

Upon dispersing powders formed by embodiments of the invention in an aqueous medium (e.g., in water at 4 °C), non-aggregated, individual hydrogel particles (beads) with sizes and shapes similar to the oil-stabilized freshly made beads are immediately re-generated. Surprisingly, as with freshly-prepared beads, beads from the powders generated by these methods can readily be crosslinked and annealed using various techniques to form microporous beaded hydrogel scaffolds. Such scaffolds have applications in a myriad of fields, including biomedical engineering, regenerative medicine, drug/gene/RNA delivery and the like.

Other objects, features, and advantages of the present invention will become apparent to those skilled in the art from the following detailed description. It is to be understood, however, that the detailed description and specific examples, while indicating some embodiments of the present invention, are given by way of illustration and not limitation.

### BRIEF DESCRIPTION OF THE DRAWINGS

Brief descriptions of the drawing are found in the text below.

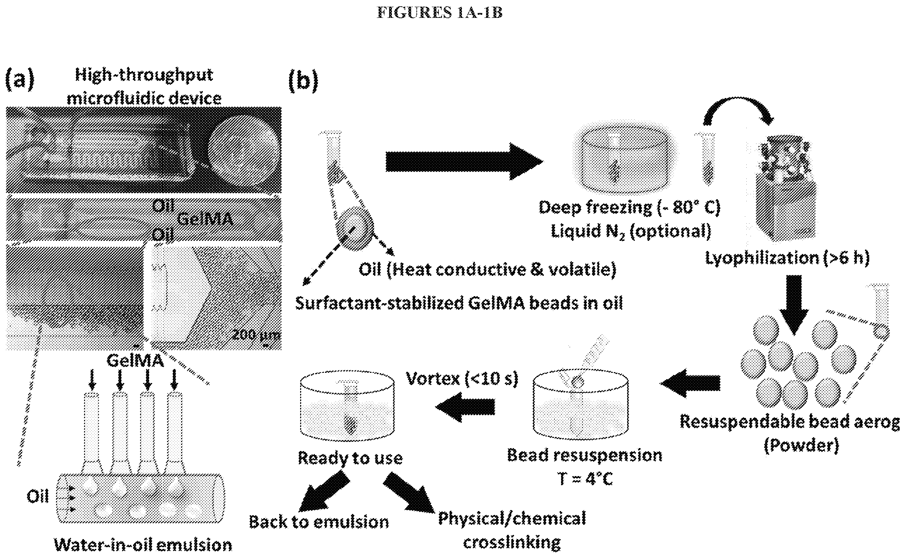

**Figures 1A and 1B** **provide a schematic showing microengineered emulsion-to-powder (MEtoP) technology to convert microfluidic-assisted hydrogel bead-oil emulsion to powders with preserved properties.** Figure 1(a) shows a high-throughput fabrication of GelMA hydrogel microbeads using a scalable microfluidic device. Emulsion-templated beads are stabilized by a mixture of heat conductive volatile oil (Novec 7500^{™}) and surfactant (PicoSurf, 0.5 wt%). The surfactant prevents the coalescence of the aqueous GelMA (dispersed) phase in the oil (continuous) phase. Figure 1(b) shows how the GelMA beads in the engineered oil phase were directly deep frozen at -80 °C, followed by lyophilization, which produced fine powders of segregated particles. The powders may readily be suspended in cold water, yielding immediately-swollen microgels with recuperated original hydrogel properties.

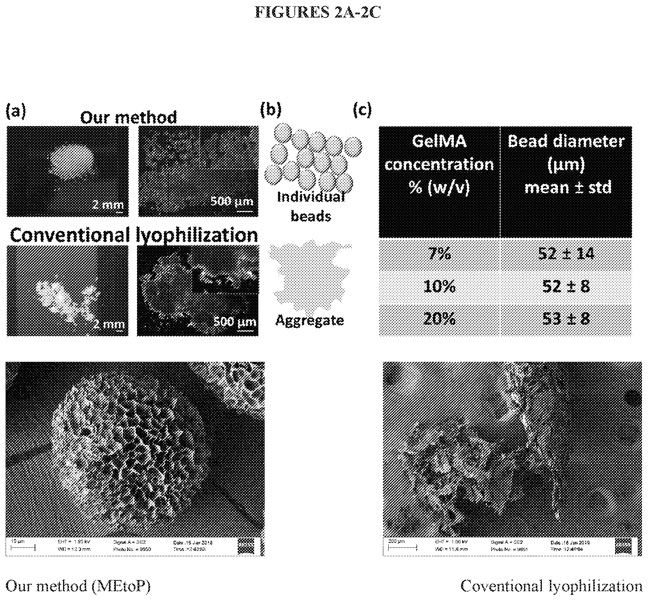

**Figures 2A-2C** **show comparisons between the powders produced via the MEtoP technology and conventional freeze-drying.** Figure 2(a) shows images (optical and scanning electron microscopy, SEM) of powders and their particles obtained from our MEtoP technology show that the particles are well segregated, whereas the powder obtained from the conventional freeze-drying of hydrogel beads in an aqueous solution yields clumped solids made up of large aggregates. Our MEtoP technology provides a protection for the dispersed phase undergoing freeze-drying, preserving the shape of individual particles. Figure 2(b) provides a schematic of individual beads produced by MEtoP technology (upper panel) compared to those yielded through conventional freeze-drying (lower panel). Figure 2(c) shows the average size of GelMA aerogel beads obtained from the MEtoP technology showing that regardless of polymer concentration, the bead size is uniform and depends only on the initial microgel size.

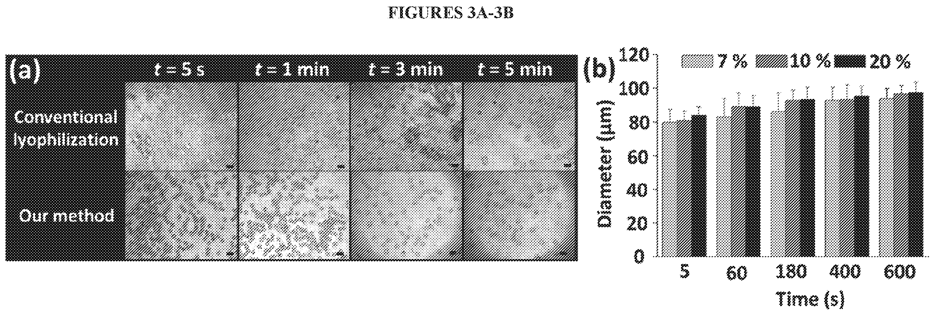

**Figures 3A-3B** **show swelling recovery of beaded GelMA powders produced via the MEtoP technology compared with the conventional method.** Figure 3(a) shows the swelling time-lapse of powder particles obtained from the MEtoP method shows the complete recovery of particle shape and size within a few minutes in cold water, whereas the freeze-dried beads in aqueous media do not swell properly, remain buckled, and often do not produce individual particles. Figure 3(b) shows the diameter of GelMA beads produced via the MEtoP method undergoing swelling in cold DI water versus incubation time. In less than 5 s, the particles swell to ~ 80% of their final diameter, showing an extremely fast recovery of the beads. Within 400 s, the particles fully swell and form microgels comparable to never-dried particles (diameter ~ 100 µm).

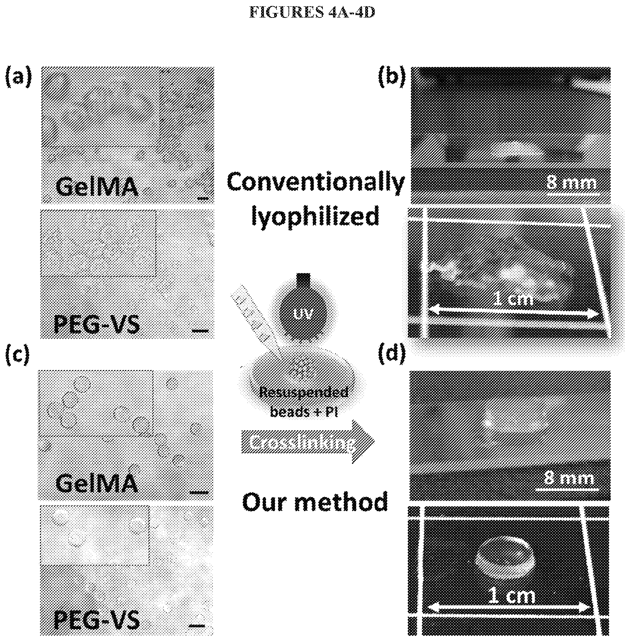

**Figures 4A-4D** **show the annealing capability of hydrogel beads (GelMA and PEGVS) prepared using the MEtoP technology or conventional lyophilization.** Figure 4(a) shows optical images of hydrogel beads prepared via the conventional method and MEtoP technology. Reswollen microgels prepared via the conventional method do not regain their original spherical shape and as shown in Figure 4(b), upon exposure to UV light in the presence of a photoinitiator (PI), they do not form a robust hydrogel construct. In contrast, as shown in Figure 4(c) hydrogel microbeads prepared by the reswelling of MEtoP-enabled powders regain their original sphericity and, as shown in Figure 4(d), undergo a robust crosslinking forming self-standing hydrogel constructs. Accordingly, our method is able not only to preserve the physical properties of the original beads, but it can also protect the chemical functionality of the beads.

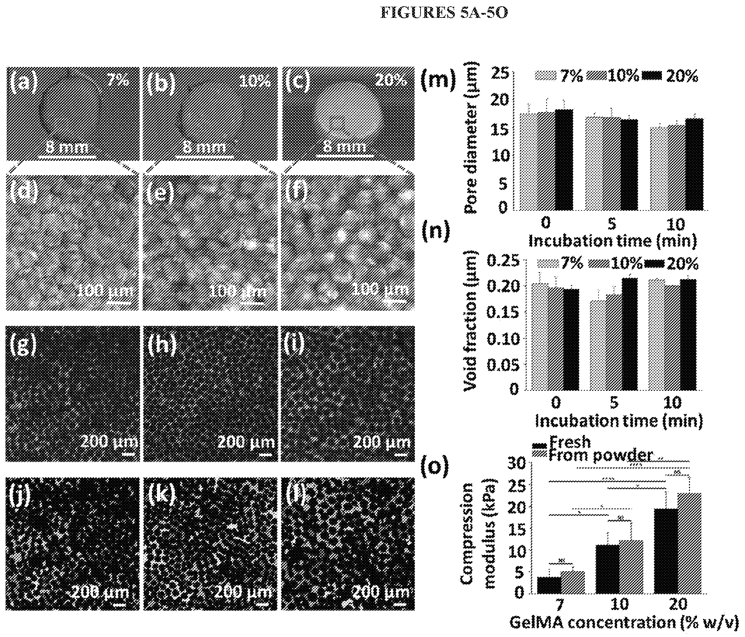

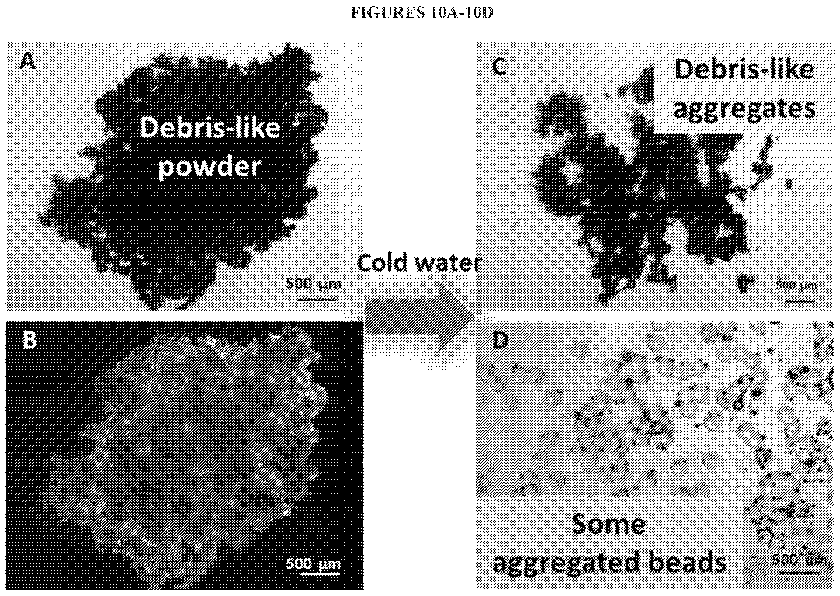

**Figure 5M-5O** **show microstructure and mechanical strength of annealed hydrogels obtained from the microengineered powders.** Self-standing hydrogels may readily be formed by annealing resuspended Figure 5(a) 7%, Figure 5(b) 10%, or Figure 5(c) 20% GelMA beads, obtained from the microengineered powders, in cold water, followed by UV light-mediated crosslinking. Bright-field images of hydrogels of 5(a), 5(b) and 5(c) are presented in panels Figure 5(d), Figure 5(e), and Figure 5(f), respectively, which show the packed, beaded structures made up of spherical beads. Two-dimensional (2D) slices of annealed beaded hydrogels obtained from confocal fluorescence microscopy show that regardless of the polymer concentrations (Figure 5(g): 7%, Figure 5(h): 10%, and Figure 5(i): 20%), the beads are able to make packed structures. The void space among beads includes a large fluorescent dextran that is unable to diffuse into the beads. Analysis of 2D slices enabled us to detect void spaces among the annealed beads with GelMA concentrations ~ 7% in Figure 5(j), 10% in Figure 5(k), and 20% in Figure 5(l), which were converted to equal area circles to extrapolate equivalent diameters. Figure 5(m) shows median pore diameter of annealed beaded GelMA scaffolds versus pre-UV exposure incubation time. The pore diameter of hydrogels is almost independent of their concentration and stiffness. Moreover, increasing the incubation time before crosslinking does not significantly affect the pore size. Figure 5(n) shows void space fraction for beaded GelMA scaffolds prepared using varying pre-crosslinking incubation time, showing that the void space is almost independent of polymer concertation (stiffness) and incubation time. Figure 5(o) shows the compression moduli of beaded scaffolds show that resuspended microengineered powders are able to yield scaffolds that are as strong as the scaffolds from freshly-prepared microgels.

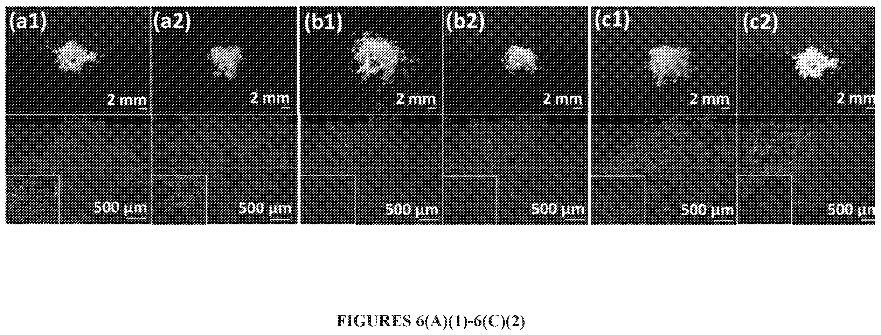

**Figure 6(A1)-6C2** **shows the effect of freeze-drying on GelMA microbeads converted to powders via the MEtoP method.** Powder GelMA obtained from microgels including Figure 6(a) 7% w/v, Figure 6(b) 10% w/v, and Figure 6(c) 20% w/v of polymer. The freezing process was conducted (1) at -80 °C followed by 5 min of liquid N₂-assisted freezing or (2) without the liquid N₂ freezing step. No difference between the samples can be observed. The freezing process starts in the aqueous phase, which may never affect the oil (at -80 °C) or be extended to the oil (at -196 °C, liquid N₂). In either case, the beads were not negatively affected by the freeze-drying process.

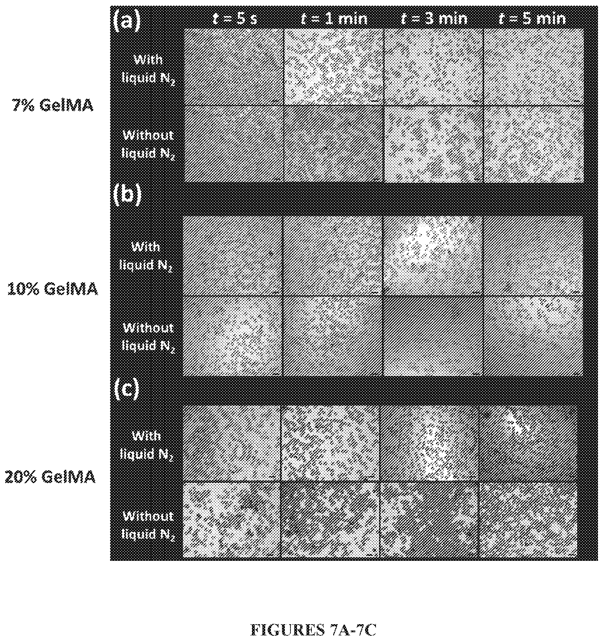

**Figures 7A-7C** **show the effect of freeze-drying on the swelling recovery of GelMA microbeads prepared via the MEtoP method.** Panels show the rehydration dynamics of powder GelMA obtained from microgels including Figure 7(a) 7% w/v, Figure 7(b) 10% w/v, and Figure 7(c) 20% w/v of polymer. The freezing process was conducted with or without liquid N₂-assisted post-freezing. In either case, the properties of beads remain unaffected by the freeze-drying process and they recuperate their shape and size comparable to freshly-prepared beads.

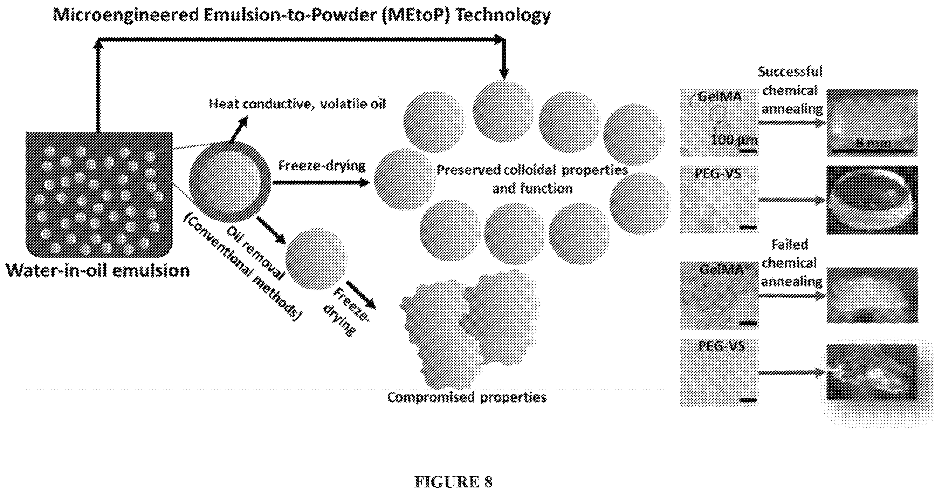

**Figure 8** **provides a general schematic of a universal high-fidelity microengineered emulsion-to-powder (MEtoP) technology.** MEtoP technology was developed to leverage the production of powders that can recuperate their original in-emulsion properties with completely-preserved molecular, colloidal, and bulk properties.

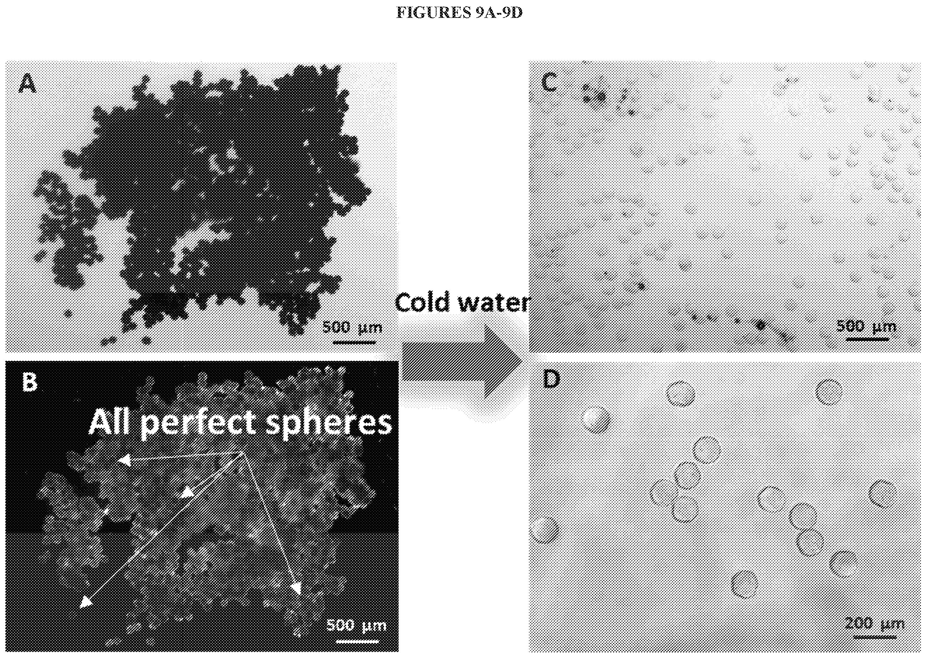

**Figures 9A-9D** show that bead generating powders (Figure 9A and 9B) readily produce stable annealable GelMA microbeads in cold water (Figure 9C and 9D).
**Figures 10A-10D** show that conventionally freeze-dried aqueous GelMA microbeads (Figure 10A and 10B) do not produce individual microbeads in cold water (Figure 10C and 10D).

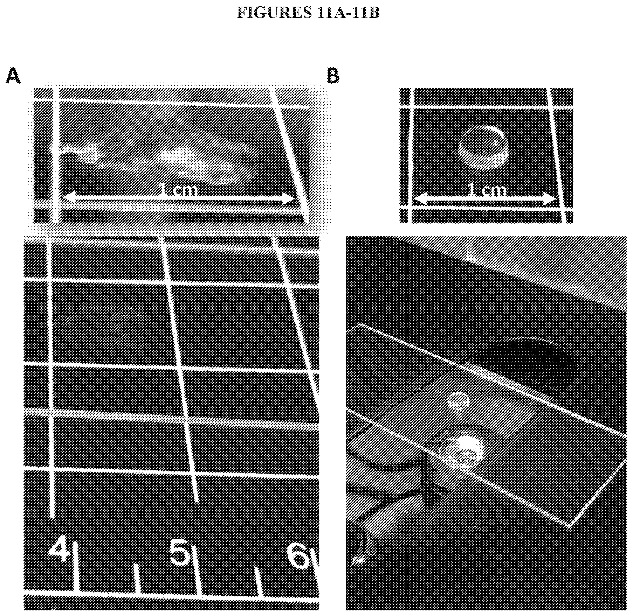

**Figures 11A-11B** **show testing the function of PEGVS microbeads produced from powders.** The inability of freeze-dried aqueous PEGVS microbeads to produce beaded hydrogel scaffolds in Figure 10(A) compared to the ability of freeze-dried emulsified PEGVS microbeads in successfully generating a beaded hydrogel scaffold in Figure 10(B).

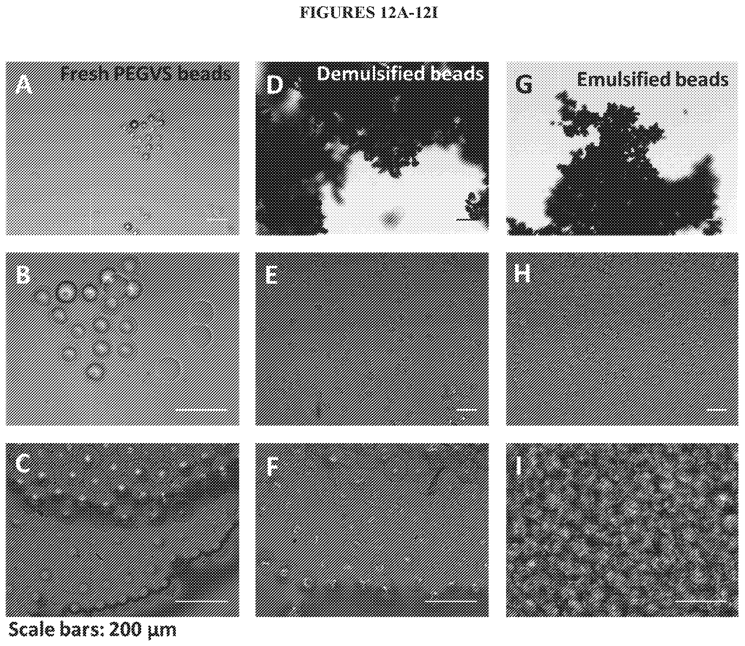

**Figures 12A-12I** **show testing the resuspension and annealing of PEGVS microbeads freshly-prepared or produced from powders.** Figures 10(A)-10(C) show freshly prepared beads in an aqueous media, dispersing and annealing well. 10(D)-10(F) show the inability of freeze-dried aqueous PEGVS microbeads in recovering their spherical shape and annealing potential. 10(G)-10(I) show the successful re-suspension, shape recovery, and annealing of freeze-dried emulsified PEGVS microbeads (i.e. following the methods disclosed herein).

### DETAILED DESCRIPTION OF THE INVENTION

Many of the techniques and procedures described or referenced herein are well understood and commonly employed using conventional methodology by those skilled in the art. In the description of the preferred embodiment, reference may be made to the accompanying drawings which form a part hereof, and in which is shown by way of illustration a specific embodiment in which the invention may be practiced. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

Converting colloidal systems, such as emulsions, dispersions, and suspensions to powders is highly demanded in a myriad of biomedical, pharmaceutical, cosmetic, oil and gas, food, energy, and environmental applications. Handling colloids is typically associated with persistent challenges including bacterial and viral contaminations, lack of terminal sterilization, impaired stability, short shelf life, high processing costs, and difficult packaging and transportation. Current techniques such as freeze-drying and spray-drying have noticeably failed in completely preserving the properties of dispersed phase while removing the continuous phase.

The invention disclosed herein provides an extremely facile method to convert colloidal systems to powders that are able to readily revive their properties upon resuspension. In our conceptually novel microengineered emulsion-to-powder (MEtoP) technology, the dispersed phase is stabilized in an engineered oil/surfactant continuous phase, followed by the deep-freezing of dispersed phase and lyophilization. The unique properties of continuous phase, including high heat conductance, low heat of vaporization, and extremely low freezing point leverage the preservation of dispersed phase physico-chemical properties. As a model system, we show that MEtoP technology successfully converts microfluidic-produced annealable microbead hydrogels to fine powders that can generate microgels with all the molecular, colloidal, and bulk characteristics of fresh beads upon resuspension in aqueous media. The universality of MEtoP technology opens new horizons for soft material processing and set the stage for the high-fidelity conversion of colloids to powders for a broad spectrum of applications. In this context, the term "colloid" is used in accordance with its art accepted meaning of a homogeneous noncrystalline substance consisting of large molecules or ultramicroscopic particles of one substance (e.g. a "lyophilizable agent") dispersed through a second substance (e.g. an aqueous solution).

The lyophilization of aqueous drops suspended in a low vapor pressure oil as disclosed herein is useful for fabricating drug aggregates/precipitates with controlled size, a material characteristic that important in a variety of biomedical applications, for example in drug delivery. The methods disclosed herein can be used to create drug-containing powders that can be inhaled and reach different regions of the airways and lungs. Inhaled micron-scale particles reach different regions of the lungs depending on size which can allow for local delivery of drugs that may otherwise have systemic effects. > 5 microns in diameter can be delivered to the pharynx or larynx, while sizes between 2-5 microns reach the trachea and bronchi, and < 2 micron particles can reach the deep lungs and alveoli. By dispersing drug of interest at a controlled concentration into aqueous drops of a known volume, artisans can control the overall mass/size of each particle. Artisans can further tune the mass/size independent of the drug concentration by adding other co-precipitate materials such as polyethylene glycol, dextran, etc. In this way, artisans can manufacture highly uniform sized drug particles for delivery without significant loss of drug to large particle aggregates or particles that are non-optimal in size. Well-controlled dry drug particles can also be used for storage of drugs in implanted devices with more uniform re-solubilization.

Similar lyophilization of gel particles can also be used for use to store and ship hydrogel particles that are used for diagnostic applications or in creating particle-based biosensors. It is desirable to have well-controlled size and shape particle-based biosensors for many applications to ensure reactions are uniform across these sensors. These particle-based biosensors can be lyophilized using the described approaches to include reagents, e.g. sensing molecules, nucleic acids, enzyme substrates, lysis reagents, reaction mixes for PCR, or other nucleic acid amplification mixes. These reagents can be loaded into the initial drops/hydrogel precursor to ensure their distribution into the matrix of the particle-based sensor and release upon rehydration.

The powders generated by the methods disclosed herein (i.e. powders that can be added to liquids to rejuvenate the original properties of the freshly-prepared liquid-based colloidal systems) find applications in a broad spectrum of industries, including but not limited to pharmaceutical companies, food industry, hygiene and personal care industries, paint industry, biomedical companies (regenerative hydrogels, drug delivery systems, peptide and protein stabilization, immunomodulating implants, etc.), and colloidal probes (imaging, sensing, diagnostics). As an example, storing uncrosslinked microfluidic-made polymeric particles in aqueous media is not trivial. These particles are typically stabilized in oil and need further purification before use to break the emulsion. The purification is typically conducted using a demulsifier, such as perfluorooctanol solution in Novec 7500^{™} oil, which may be expensive and toxic. Furthermore, before breaking the emulsion, the polymeric beads must typically be crosslinked to hold their structure in water. To the best of our knowledge, currently, no method is available to convert annealable microbeads into powders that can readily be re-suspended and revived without significant damage or aggregation. These powders may eliminate the necessity of using freshly-made microbeads that demand microfluidic setups or the storage and shipment in a hydrated state. Consequently, this technology will enable artisans to provide the building blocks of a newly-emerged family of microporous hydrogels (beaded hydrogels) worldwide.

The invention disclosed herein has a number of advantageous applications. For example, embodiments of the invention provide artisans with an enhanced ability to perform gamma or other terminal treatment/sterilization processes. Embodiments of the invention also provide artisans with an enhanced ability to decrease the risk of microbiological contamination (which is common in aqueous media). Embodiments of the invention also provide artisans with an enhanced ability to reduce the rate of hydrolysis-driven or other types of degradation. Embodiments of the invention also provide artisans with an enhanced ability to increase product shelf-life and physico-chemical stability. Embodiments of the invention also provide artisans with an enhanced ability to preserve pharmacological activity of drugs and other cargos embedded in the beads. In these ways, embodiments of the invention can save processing energy and cost of various materials as well as facilitating the shipment of such materials.

As illustrated by the schematic shown in Figure 9, we show how our technology can be used to produce powders that can instantly generate uniform-sized gelatin methacryloyl (GelMA) microbeads upon dispersing in cold water. The beads are then crosslinked and annealed to each other using UV light, yielding a beaded-scaffold with similar properties to a scaffold that is made from never-freeze-dried, freshly purified microbeads. This is the first experimental demonstration of annealable microbead producing powders. As shown in Figure 10, when the purified, cold water-stabilized microbeads are frozen in an aqueous medium followed by lyophilization, the resulting powder does not produce individual beads upon re-suspension in cold water. Instead, this method yields damaged and permanently aggregated particles, which do not resemble the freshly-prepared microbeads. This experiment shows the importance of our novel method for processing microfluidic-made microbeads to provide a revivable dry bead platform.

As shown in Figure 11 and 12, embodiments of the invention provide universal methods to convert a variety of materials, such as multi-armed poly(ethylene) glycol-vinyl sulfone (PEGVS), into bead-producing powders. Embodiments of the invention can also be used to convert other emulsified systems, such as emulsions, microemulsions and nanoemulsions into stable powders with more uniform particle size. These emulsions can contain organic molecules that crystalize or precipitate (such as drugs or biomolecules, DNA, proteins, etc.) or inorganic molecules that can aggregate or form controlled clusters (such as metal ions, metal nanoparticles, salts, etc.). For example, controlled clusters of metallic nanoparticles can have unique optical or plasmonic properties.

Disclosed herein are methods of making a lyophilized composition from lyophilizable agent dispersions, suspensions, emulsions, and the like. These methods comprise combining together an oil, a surfactant, a lyophilizable agent (e.g. beads, particles and the like) and an aqueous solution so as to form a multiphase system comprising the lyophilizable agent dispersed within an aqueous phase that is stabilized within a continuous oil/surfactant phase. These methods further include freezing this multiphase system; and then removing water, oil and surfactant from the multiphase system using a drying process so that the lyophilized composition is made. In typical embodiments of the invention, the drying process comprises a vacuum mediated drying process and/or a heat mediated drying process. In illustrative embodiments, the lyophilizable agent dispersed within an aqueous phase comprises suspensions, colloidal dispersions and the like. In certain embodiments of the invention, the lyophilizable agent dispersed within an aqueous phase comprises the lyophilizable agent dispersed within droplets of an aqueous solution. Illustrative embodiments of such methods are described in Figures 1 and 8 and in the examples below.

The oil and surfactant are selected to have certain material properties such as a low heat of vaporization/sublimation. The oil (and/or the surfactant) is selected to exhibit a boiling point at 1 atmosphere that is above 50 °C (or at least above 100°C), and/or a pour point that is at least above -50°C (or at least above -100°C). Typically in the methods of making a lyophilized composition, the multiphase system is cooled to at least about 0 °C, least about -78 °C, or at least about -195°C. In some embodiments of the invention, the lyophilizable agent comprises hydrogel particles, and resuspension of the lyophilized composition in an aqueous resuspension solution generates predominantly (i.e. 51% to over 99%) non aggregated hydrogel particles. In certain embodiments of the invention, microporous hydrogels formed from resuspended lyophilized hydrogel particles exhibit compression moduli that are at least 75%, 80%, 85% or 90% the compression moduli observed in a microporous hydrogel formed from control hydrogel particles that have not been lyophilized.

A wide variety of lyophilized compositions may be made by the methods disclosed herein. Such lyophilized compositions can comprise beads, particles, polymer precursors, polymers, polynucleotides, polypeptides or the like having a variety of desirable properties. For example, in certain embodiments of the invention that lyophilize hydrogel particles, the method yields undamaged and unaggregated particles having the hydrogel properties of freshly-prepared microbeads. In certain embodiments of the invention, the lyophilized composition forms a powder comprising monodispersed particles having a standard deviation of <10%, 20% or 30% in size.

The invention also includes methods of making a resuspended lyophilized composition. These methods comprise combining a lyophilized composition with an aqueous resuspension solution such that a resuspended lyophilized composition is made. In these methods, the lyophilized composition that is resuspended is made by the method of any one of claims 1 to 9 comprising combining together an oil, a surfactant, a lyophilizable agent and an aqueous solution so as to form a multiphase system comprising the lyophilizable agent dispersed within an aqueous phase that is stabilized within a continuous oil/surfactant phase; freezing this multiphase system; and then removing water, oil and surfactant from the multiphase system using a drying process so that the lyophilized composition is made. In certain embodiments of the invention, the aqueous resuspension solution comprises a crosslinking agent. In some embodiments of the invention, the lyophilizable agent comprises hydrogel particles. In such methods, artisans can further crosslink the hydrogel particles so as to form a microporous hydrogel scaffold. In certain embodiments of the invention, microporous hydrogel scaffold made by these methods exhibits a compression modulus that is at least 80% the compression moduli observed in a control microporous hydrogel formed from identical hydrogel particles that have not been lyophilized.

In illustrative working embodiments of these methods that are disclosed herein, the lyophilizable agent comprises hydrogel particles and the aqueous resuspension solution comprises a crosslinking agent. In such methods where the lyophilizable agent comprises hydrogel particles, the method can further entail crosslinking the hydrogel particles so as to form a microporous hydrogel scaffold. In some of these embodiments of the invention, a microporous hydrogel scaffold can be formed from the resuspended lyophilized composition. Typically in these embodiments of the invention, the microporous hydrogel scaffold exhibits a compression modulus that is at least 80% the compression modulus observed in a control microporous hydrogel formed from identical hydrogel particles that have not been lyophilized.

### FURTER ILLUSTRATIVE ASPECTS OF THE INVENTION

As noted above, the invention disclosed herein provides a novel, facile technology for the on-demand microengineering of emulsions to convert the dispersed phase to a fine powder with completely-preserved molecular, colloidal, and bulk properties. The microengineered emulsion-to-powder technology (MEtoP) hinges on protecting the dispersed phase undergoing harsh deep-freezing and low-pressure lyophilization via engineering the aqueous droplet-oil interface using a heat-conductive, volatile, and low-freezing-point oil. We examine two model systems, gelatin methacryloyl (GelMA) and PEG-vinylsulfone (PEGVS), which were converted to a uniformly-sized droplets-in-oil emulsion, followed by physical/chemical crosslinking to form hydrogel microbeads. We show that in contrast to conventional lyophilization, which produced permanently-clumped polymer aggregates with lost functionality, the MEtoP technology is, for the first time, able to convert the microgels into powders with extremely fine particles that can be successfully redispersed in an aqueous medium and recuperate their shape, size, and chemical functionality with a very high fidelity. Accordingly, this is the first report of powders that can produce annealable microbeads with properties identical to freshly-prepared off-the-microfluidic device microgels.

### ILLUSTRATIVE MATERIALS

Silicon wafers were purchased from University Wafer (MA, USA), negative photoresist was from KMPR 1050, MicroChem Corp. (MA, USA), and the microfluidic chips were fabricated using polydimethylsiloxane (PDMS) base/the curing agent (SYLGARD^{™} 184 Elastomer Kit, Dow Coming, MI, USA). The microfluidic tubing was 1569-PEEK Tubing Orange 1/32" OD x .020" ID (IDEX Corp., IL, USA) and Tygon Flexible Plastic Tubing 0.02" ID x 0.06" OD (Saint-Gobain PPL Corp., CA, USA). The microfluidic device was treated with Aquapel^{®} Glass Treatment (Pittsburgh Glass Works LLC, PA, USA). 3M^{™} Novec^{™} 7500 Engineered Fluid (Novec 7500 oil) was purchased from 3M (MN, USA). Photoinitiator 2-hydroxy-1-(4-(hydroxyethoxy)phenyl)-2-methyl-1-propanone (Irgacure 2959), gelatin from porcine skin (type A, 300 bloom), methacrylic anhydride (MA, 94%), 1H,1H,2H,2H-perfluoro-1-octanol (97%), and fluorescein isothiocyanate (FITC)-dextran (500 kDa) were provided by Sigma-Aldrich (MO, USA). Milli-Q water (electrical resistivity ~ 18.2 MΩ cm at 25 °C) was from Millipore Corporation. Dialysis membranes (molecular weight cutoff ~ 12-14 kDa) were purchased from Spectrum Lab Inc (CA, USA). Cover slips (No. 1) and VistaVision^{™} Microscope Slides (Plain 3" x 1") were provide by VWR (PA, USA), and microscope glass slides (18 mm x 18 mm x 300 µm) were purchased from Fisher Scientific (PA, USA). Pico-Surf^{™} 1 (5% (w/w) in Novec^{™} 7500) was provided by Sphere Fluidics Inc (Cambridge, UK). Dulbecco's phosphate-buffered saline (DPBS) solution (1X) was from Gibco (NY, USA). Four-arm poly(ethylene) glycol (Mw ~ 20,000)-vinylsulfone (PEG -VS) was from NOF Corporation, and dithiothreitol (DTT), triethanolamine, triethylamine, and Eosin Y were purchased from Sigma-Aldrich.

### ILLUSTRATIVE METHODS

**Microfluidic device fabrication:** To generate uniform-sized spherical microbeads, a high-throughput microfluidic water-in-oil emulsion method (42-44) was modified and used. Highly parallelized step emulsification devices were fabricated as previously reported (45) using standard soft lithography techniques. Master molds were fabricated using a two-layer photolithography process. Mechanical grade silicon wafers (4 in) were sequentially layered with photoresist (32 um KMPR 1025, 160 um KMPR 1050) and patterned using standard photolithography techniques. At a ratio of 10 to 1, the PDMS base and the curing agent were mixed and poured onto the molds affixed to petri dishes, followed by degassing and curing in an oven (65 °C for >4 h). The PDMS device was detached from the mold and perforated (0.8 mm holes) at the inlets and outlets. To seal the microchannels, both the device and a glass slide were activated via air plasma for 40 s (Plasma Cleaner, Harrick Plasma, NY, USA) and bonded together. To render the channel surfaces fluorophilic, the device was treated with Aquapel, followed by washing with the Novec 7500^{™} oil.

**GelMA synthesis:** Gelatin type A was modified with a high degree of methacryloyl substitution to synthesize GelMA following our previous protocols (5,6). Briefly, 10 g of gelatin was dissolved in 100 mL of warm DPBS (50 °C), followed by the dropwise addition of 8 mL of MA while stirring the solution at 240 rpm. This resulted in a turbid biphasic mixture, which was allowed to react by stirring for 2 h at 50 °C. This condition prevents protein hydrolysis (6,7). Upon completion of reaction duration, excessive DPBS was added to the mixture to stop the reaction. The mixture was then loaded in the dialysis membranes and stirred in DI water (40 °C) for at least seven days to remove methacrylic acid and other impurities. The result of dialysis was a clear GelMA solution, which was lyophilized and stored in room temperature before using for microgel fabrication.

**GelMA bead fabrication:** Freeze-dried GelMA was dissolved in a mixture of DPBS and the photoinitiator (0.5% w/v, Irgacure 2959) at 80 °C for ~20 min to yield GelMA solutions (7-20 % w/v). These solutions provided the dispersed (aqueous) phase in the high-throughput microfluidic device, which were injected in the pinching flow of Novec 7500 oil-surfactant (0.5 wt% PicoSurf) mixture, simultaneously introduced into the microfluidic device using syringe pumps (Harvard Apparatus PHD 2000, MA, USA) to form surfactant-stabilized 100 µm beads of GelMA in the engineered oil (continuous) phase. The microfluidic setup including the syringes and tubing were maintained at 37-40 °C to avoid GelMA sol-gel transition and device blockage. A microcentrifuge tube was used to collect the bead suspension in oil and store at 4 °C.

**PEG-VS bead fabrication:** PEG-VS microgel beads were fabricated as previously reported (45). Briefly, a gel precursor solution composed of 5 wt% of PEG-VS and 8 mM DTT in 300 mM triethanolamine (pH ~ 5) was prepared. The precursor solution was injected into a parallelized step-emulsification device along with an oil phase (Novec 7500^{™} including 0.5% PicoSurf) at a flow ratio of 2:1 to generate water droplets-in-oil. An oil phase composed of Novec 7500^{™} oil and 3% triethylamine was introduced downstream to increase the pH in the droplets to 8.2 to deprotonate the thiol groups on the DTT molecules and induce gelation of the PEG-VS microbeads via Michael addition reaction. The beads were collected and incubated overnight at room temperature.

**Microengineered emulsion-to-powder (MEtoP) technology:** MEtoP technology is based on protecting the dispersed phase of an emulsion to preserve its physical and chemical cues during harsh freezing and lyophilization procedures. The powders produced via the MEtoP technology can recuperate their in-emulsion and/or in-solution properties upon rehydration. In this technology, the interface of dispersed (aqueous) phase is stabilized using an engineered oil (e.g., Novec^{™} 7500) including a surfactant (e.g. Pico-Surf^{™}). The oil is highly heat conductive, volatile, and has a low freezing point. The oil/surfactant-stabilized aqueous phase is deep frozen (e.g., at -80 °C and/or -196 °C) and transferred to a lyophilizer (Labconco FreeZone Benchtop Freeze Dry System) to sublimate the ice and remove the volatile oil under vacuum (e.g., 0.06 mbar) for at least 6 h. This process results in a one-step conversation of emulsions to powders made up of microengineered particles with preserved properties.

**Conventional lyophilization of hydrogel microbeads:** The hydrogel microbead-in-oil emulsions were pulse centrifuged (6300 rpm, 10 s, GmCLab mini centrifuge, Gilson, France), and the excess oil was removed using a pipette. To break the emulsion, a perfluorooctanol solution (20%) in Novec^{™} 7500 oil was added to the bead suspension (1:1 volume ratio), which removed the surfactant. The GelMA beads were always maintained at 4 °C and the PEG-VS beads were at 25 °C. The suspension was diluted in a DPBS solution, and the physically-crosslinked (GelMA) or chemically-crosslinked (PEG-VS) microbeads were pulse centrifuged and the supernatant was removed. The beads were transferred to another microcentrifuge tube using a positive-displacement pipette (MICROMAN^{®} E, Gilson, WI, USA) and always maintained in DPBS. The tubes were frozen at -80 °C overnight, followed by lyophilization at ~0.06 mbar bar for at least 24 h. The process was conducted in a way that the frozen samples never melted and always remained frozen until competing the ice sublimation process.

**Beaded hydrogel powder imaging and rehydration:** Powders were transferred onto a cover gas and imaged using a camera (Axio cam 503 mono, 60N-C 1" 1,0X). The microstructures of powders were visualized using dark or bright filed microscopy (Axio Observer 5, Zeiss, Germany). The rehydration (swelling) of powder GelMA beads was investigated by suspending them in cold DPBS (1X, 4°C). Similar experiment was conducted with PEGVS at room temperature. Brightfield microscopy at predefined time intervals was conducted to image the beads and measure their size via image analysis using ImageJ (Version 1.52e, National Institute of Health, USA).

**Fabrication of beaded GelMA (B-GelMA) scaffolds:** To evaluate the properties of beads produced using the MEtoP technology and compare them with the freshly-prepared beads as well as the conventionally-lyophilized ones, the oil-free microbeads were suspended in DPBS (4 °C) containing the photoinitiator (0.5% w/v, Irgacure 2959), followed by pulse centrifugation for 10 s to pack the beads at the bottom of container. Using a positive displacement pipette, the concentrated microbead suspension was transferred into a PDMS mold (diameter ~ 8 mm, height ~ 1 mm) and incubated for a desired period (to investigate the effect of packing), followed by UV light (360-480 nm, Omnicure, Excelitas, CA, USA) exposure (intensity ~ 10 mW cm⁻²) for 2 min, yielding chemically-crosslinked and possibly annealed microbeads.

**Fabrication of beaded PEG-VS scaffolds:** PEG-VS microbeads were incubated in a buffer solution (HEPES, pH ~ 7.4, including 10 mM CaCl₂) containing a white light photoinitiator (Eosin Y, 10 µM) for 1 h. Beads were concentrated via centrifugation and injected into a PDMS mold using a positive displacement pipette. Beads were covalently linked together to form an interconnected microporous scaffold by exposing the sample to white light (V-Lux 1000) for 3 min.

**Pore size analysis:** Chemically-crosslinked hydrogel scaffolds were incubated in a FITC-dextran solution (15 mM) to fill the void space in the scaffolds with the dye. The large molecular size of dextran prevents its diffusion into the beads, enabling us to visualize the void spaces. The dye-infused scaffolds were imaged using a Leica inverted SP5 confocal microscope (Germany) at the California NanoSystems Institute (CNSI). For each sample, 77 z-slices were captured to cover total height of ~150 µm, and at least 3 samples per condition were analyzed. Median pore diameter and void space fraction were measured using a custom-developed Matlab code (Matlab, version 2017b). Briefly the code converted the stacked images into discrete regions using an adaptive thresholding, and the void space fraction was calculated based on the voxel volume of void space regions. Average pore diameter was calculated based on a previously-published method (8).

**Mechanical analyses (compression tests):** Crosslinked beaded GelMA samples (UV intensity ~ 10 mW cm⁻², exposure time ~ 2 min) in PDMS molds (diameter ~ 8 mm and height ~ 1 mm) were transferred to the Instron mechanical tester (Instron 5542, Norwood, MA, USA) and compressed at a rate ~ 1 mm min⁻¹. The linear stress-strain region was fitted with the best line (0-10% strain) and the slope was registered as the compression modulus (= stress/strain).

**Statistical analysis:** Measurements were performed at least in triplicate. The data were reported as mean values ± standard deviation. The one-way analysis of variance (ANOVA) was carried out followed by Tukey's multiple comparisons. Statistically significant differences were identified when p-values were lower than 0.05 (*p<0.05), 0.01 (**p<0.01), 0.001 (***p<0.001), and 0.0001 (****p<0.0001).

### RESULTS AND DISCUSSION

The principles of MEtoP technology is based on protecting the dispersed phase of an emulsion during harsh freezing and lyophilization steps using an engineered oil that (i) has a decent heat conductivity, (ii) is easily removed under vacuum during lyophilization, (iii) preferably has a low freezing point, and (iv) is well mixed with a surfactant to stabilize the oil-water interfaces. The heat conductivity of the oil (continuous phase) facilitates the deep freezing of dispersed phase, while the oil remains unfrozen, followed by the vacuum-mediated removal during lyophilization. We selected the mixture of Novec^{™} 7500 oil with a surfactant (PicoSurf, 0.5 wt%), which satisfies all of our design criteria. Many other oils can potentially be used for this purpose. To evaluate the capability of MEtoP technology in converting an aqueous dispersed phase to a powder with preserved molecular, colloidal, and bulk properties, the uniformly-sized microspheres of hydrogels (e.g., GelMA) were produced using a high-throughput step emulsification microfluidic device, shown in **Figure 1a****,** in which varying concentrations of GelMA dissolved in an aqueous solution were injected along with the engineered oil mixture (continuous phase) to generate hydrogel microbeads with a diameter of ~100 µm. Droplet generation is driven by a sudden expansion at the end of each of the droplet generation channels. As the dispersed phase reaches the end of the channel, the expansion induces an instability that drives uniform drop formation (44). The surfactant-stabilized hydrogel beads were deep-frozen at -80 °C as presented in **Figure 1b****,** leaving the oil phase that has a pour (melting) point of ~ -100 °C liquid. The partially-frozen samples were transferred to a lyophilizer and maintained under vacuum for at least 6 h. We have also tried to freeze the oil using the liquid N₂, which resulted in the formation of all-frozen samples undergoing oil melting within a few minutes during lyophilization. Regardless of the freezing method, the MEtoP process results in the formation of fine powders that can readily be resuspended or converted to their original emulsion state.

**In** **Figure 2****,** the powder generated using the MEtoP technology is compared with the powder produced by freeze-drying the GelMA beads in the aqueous phase (conventional lyophilization). In macro-scale, the powder that was produced using the MEtoP technology had very fine particles as shown in **Figure 2a****,** whereas the conventional lyophilization yielded large aggregated clumps. At the micro-scale, the dark field optical images of powders show that the microengineered powder was made up of extremely fine, segregated particles while the conventional method resulted in the aggregated particles often difficult to be individually distinguish. The products of these two methods are schematically shown in **Figure 2b****.** The particle size of bead powders including 7%, 10%, and 20% of GelMA, presented in **Figure 2c****,** shows that independent of the biopolymer concentration, the dried beads have an average particle diameter of ~ 50 µm. This is almost half of the freshly-prepared beads as a result of water removal and biopolymer shrinkage. Images of GelMA (7-20%) powders and their constituent beads are presented in **Figure 6****.** As can be seen in this figure, there is no significant difference between the powders prepared via an extended liquid N₂ freezing compared to the ones that were only frozen at -80 °C. Accordingly, even the temporary freezing of oil does not have any effect on the outcome of MEtoP technology, attesting to its robustness in producing fine, segregated beads.

To assess the capability of lyophilized powders in recuperating their original shape and size, the time evolution of their diameter upon resuspension in DPBS was measured using optical microscopy. **Figure 3** shows that the dry microbeads produced via the MEtoP method swell to ~80% of their original (in-emulsion) size almost immediately after introducing them into the aqueous phase. The swelling of the microengineered beaded powder completes in less than 10 min, resulting in fully swollen beads with sizes similar to the freshly-prepared beads **(****Figure 3b****).** In the contrary, the powder produced via the conventional method does not yield individual beads and remain permanently aggregated in the aqueous medium. This attests to the promising potential of MEtoP in providing an infrastructure for preserving the macro- and microscale properties of lyophilized substances. Images of the rehydration of beads produced via the MEtoP technology (with or without the liquid N₂ freezing step) are presented in **Figure 7****.** As can be seen in this figure, the beads regain their original shape and size within a few minutes regardless of the freezing method and biopolymer concentration.

The preservation of chemical cues in photoactive hydrogels, such as GelMA and PEG-VS, during MEtoP processing was compared with the hydrogels underwent the conventional lyophilization method. To this end, hydrated powders, shown in **Figure 4a****,** were resuspended in a solution of DPBS including a UV-active photoinitiator. The resuspended beads were exposed to UV light to initiate the chemical crosslinking of methacryloyl or vinylsulfone groups. **Figure 4b** shows the crosslinked scaffolds post-UV light exposure. The scaffolds that were constructed from the beads produced via the conventional method were not able to hold their shape due to the lack of effective bead-bead chemical conjugation. Interestingly, the crosslinking of beads prepared via the MEtoP method formed self-standing hydrogel constructs, which implies that the photoactivated bead-bead annealing has taken place successfully.

The properties of annealed scaffolds were evaluated to investigate the differences between the hydrogels prepared from the MEtoP beads compared to the freshly-prepared microgels. **Figure 5** presents the macro-scale and micro-scale properties of the annealed scaffolds. Optical images of annealed GelMA scaffolds made up of MEtoP beads including 7% **(****Figure 5a****),** 10% **(****Figure 5b****),** and 20% **(****Figure 5c****)** GelMA show that even at low concentrations of GelMA (i.e., soft beads), MEtoP beads are able to form a self-standing hydrogel construct. At the microscale, all these hydrogels are made up of annealed microspheres that form a packed structure with permanent micropores **(****Figure 5d****-f).** Infusing a large fluorescent dye (FITC-dextran) in the pores of hydrogels enabled us to image layers of these constructs. **Figures 5g**,**h**,**i** show examples of z-stacks obtained from confocal microscopy of hydrogels constructed from MEtoP GelMA beads with 7%, 10%, and 20% biopolymer, respectively. The images were processed using a custom-built Matlab code in which the fluorescent-labeled spaces were filled with varying diameters of spheres. The processed void spaces (examples shown in **Figures 5j****-l**) from 70 stacks (height ~150 µm) provided the median pore diameter and void space fraction of annealed hydrogels. **Figure 5m** presents the median pore dimeter of hydrogels prepared via crosslinking the MEtoP beads (7-20 %) immediately, after 5 min, or after 10 min post-transferring to a PDMS mold. Regardless of the GelMA concentration, all the hydrogel samples attain a similar median pore diameter, which is almost independent of the incubation time. The pore dimeter of hydrogels fabricated from freshly-prepared GelMA microbeads is ~ 20 µm (30), which is almost identical to the MEtoP beaded gels. The median pore diameters of beaded scaffolds constructed from the freshly-prepared beads were similar to the MEtoP-based scaffolds (~18±2 µm). **Figure 5n** shows the void space fraction of annealed hydrogels. Similar to the pore dimeter, the void space is neither affected by the GelMA concentration nor it varies with the incubation time. Such an excellent similarity between the MEtoP-based beaded hydrogels and the freshly-prepared ones attests to the unique capability of MEtoP in preserving the original properties of colloidal particles and macromolecules undergoing harsh lyophilization.

To evaluate the strength of hydrogel constructs, compression tests were conducted. **Figure 5o** presents the compression modulus of annealed beaded hydrogels fabricated from MEtoP (powder) or freshly-prepared beads. Increasing the biopolymer concentrating increases the compression modulus; however, there is no significant difference between the compression modulus of beaded hydrogels fabricated from the fresh or MEtoP beads. The independency of porosity and void fraction has been shown for the microporous beaded GelMA hydrogels prepared from fresh beads (30). **Figures 5m****-o** show that the MEtoP technology can provide powders that can generate swollen microbeads with identical physical and chemical properties to the freshly-prepared, never dried microgels.

In summary, preserving the physical and chemical properties of macromolecules and colloids post-drying is of utmost importance in a broad range of pharmaceutical, biomedical, food, oil and gas processing, and energy production and storage applications. Common methods for converting a dispersed aqueous phase to a solid typically involve harsh freeze- or spray-drying steps, which often result in the loss of original properties. Here, we introduce a facile method to prepare powders that can readily redisperse and recuperate the original properties of their wet state. We have invented a method called the MEtoP (microengineered emulsion-to-powder) technology involving the stabilization of a dispersed phase using an engineered oil, which acts as a protective layer and preserves the properties of dispersed phase during lyophilization. The engineered oil phase is heat conductive and can readily be evaporated, which provides a physical barrier among the dispersed phase components while permitting proper heat and mass transfer. For the first time, we show that, within a few hours, emulsions can be readily converted to powders that can be resuspended and gain their original properties. As a model system, we show that MEtoP was able to convert functionalized gelatin (GelMA) and PEG (PEG-VS) hydrogel microbeads into finely-separated spherical particles that regain their physical and chemical properties within minutes post-suspension. The MEtoP technology may pave the way for large-scale, safer, more cost-effective, and universal emulsion conversion to powders, enabling gamma or other terminal treatment/sterilization processes, decreasing the risk of microbiological contamination, reducing the rate of hydrolysis-driven or other types of degradation, and increasing shelf-life, physico-chemical stability, and pharmacological activity of substances while facilitating the shipment and decreasing processing energy and cost.

### CONCLUSION

This concludes the description of the preferred embodiment of the present invention. The foregoing description of one or more embodiments of the invention has been presented for the purposes of illustration and description. It is not intended to be exhaustive or to limit the invention to the precise form disclosed.

### References

(1) Y. Xia, X. Na, J. Wu, G. Ma, The Horizon of the Emulsion Particulate Strategy: Engineering Hollow Particles for Biomedical Applications, Adv. Mater. (2018) 1801159.
(2) W. Li, L. Zhang, X. Ge, B. Xu, W. Zhang, L. Qu, C.-H. Choi, J. Xu, A. Zhang, H. Lee, Microfluidic fabrication of microparticles for biomedical applications, Chem. Soc. Rev. 47 (2018) 5646-5683.
(3) S.N. Kale, S.L. Deore, Emulsion micro emulsion and nano emulsion: A review, Syst. Rev. Pharm. 8 (2017) 39.
(4) P. Shah, D. Bhalodia, P. Shelat, Nanoemulsion: a pharmaceutical review., Syst. Rev. Pharm. 1 (2010).
(5) S.F. Wong, J.S. Lim, S.S. Dol, Crude oil emulsion: a review on formation, classification and stability of water-in-oil emulsions, J. Pet. Sci. Eng. 135 (2015) 498-504.
(6) D.J. McClements, Food emulsions: principles, practices, and techniques, CRC press, 2015.
(7) L. Mao, Y.H. Roos, C.G. Biliaderis, S. Miao, Food emulsions as delivery systems for flavor compounds: A review, Crit. Rev. Food Sci. Nutr. 57 (2017) 3173-3187.
(8) M.N. Yukuyama, D.D.M. Ghisleni, T.J.A. Pinto, N.A. Bou-Chacra, Nanoemulsion: process selection and application in cosmetics-a review, Int. J. Cosmet. Sci. 38 (2016) 13-24.
(9) O. Sonneville-Aubrun, M.N. Yukuyama, A. Pizzino, Application of Nanoemulsions in Cosmetics, in: Nanoemulsions, Elsevier, 2018: pp. 435-475.
(10) R.G. Larson, The structure and rheology of complex fluids (topics in chemical engineering), Oxford Univ. Press. New York• Oxford. 86 (1999) 108.
(11) S.-Y. Teh, R. Lin, L.-H. Hung, A.P. Lee, Droplet microfluidics, Lab Chip. 8 (2008) 198-220.
(12) R. Seemann, M. Brinkmann, T. Pfohl, S. Herminghaus, Droplet based microfluidics, Reports Prog. Phys. 75 (2011) 16601.
(13) C. Martino, A.J. deMello, Droplet-based microfluidics for artificial cell generation: a brief review, Interface Focus. 6 (2016) 20160011.
(14) L. Shang, Y. Cheng, Y. Zhao, Emerging droplet microfluidics, Chem. Rev. 117 (2017) 7964-8040.
(15) R. Riahi, A. Tamayol, S.A.M. Shaegh, A.M. Ghaemmaghami, M.R. Dokmeci, A. Khademhosseini, Microfluidics for advanced drug delivery systems, Curr. Opin. Chem. Eng. 7 (2015) 101-112.
(16) Z. Liu, N. Banaei, K. Ren, Microfluidics for combating antimicrobial resistance, Trends Biotechnol. 35 (2017) 1129-1139.
(17) L.A. Bawazer, C.S. McNally, C.J. Empson, W.J. Marchant, T.P. Comyn, X. Niu, S. Cho, M.J. McPherson, B.P. Binks, F.C. Meldrum, Combinatorial microfluidic droplet engineering for biomimetic material synthesis, Sci. Adv. 2 (2016) e1600567.
(18) X. Hou, Y.S. Zhang, G. Trujillo-de Santiago, M.M. Alvarez, J. Ribas, S.J. Jonas, P.S. Weiss, A.M. Andrews, J. Aizenberg, A. Khademhosseini, Interplay between materials and microfluidics, Nat. Rev. Mater. 2 (2017) 17016.
(19) K.S. Elvira, X.C. i Solvas, R.C.R. Wootton, The past, present and potential for microfluidic reactor technology in chemical synthesis, Nat. Chem. 5 (2013) 905.
(20) H. Song, D.L. Chen, R.F. Ismagilov, Reactions in droplets in microfluidic channels, Angew. Chemie Int. Ed. 45 (2006) 7336-7356.
(21) O.J. Dressler, X. Casadevall i Solvas, A.J. deMello, Chemical and biological dynamics using droplet-based microfluidics, Annu. Rev. Anal. Chem. 10 (2017) 1-24.
(22) C.W. Shields IV, C.D. Reyes, G.P. López, Microfluidic cell sorting: a review of the advances in the separation of cells from debulking to rare cell isolation, Lab Chip. 15 (2015) 1230-1249.
(23) J.R. Heath, A. Ribas, P.S. Mischel, Single-cell analysis tools for drug discovery and development, Nat. Rev. Drug Discov. 15 (2016) 204.
(24) Z. Zhu, C.J. Yang, Hydrogel droplet microfluidics for high-throughput single molecule/cell analysis, Acc. Chem. Res. 50 (2016) 22-31.
(25) S. Nagelberg, L.D. Zarzar, N. Nicolas, K. Subramanian, J.A. Kalow, V. Sresht, D. Blankschtein, G. Barbastathis, M. Kreysing, T.M. Swager, Reconfigurable and responsive droplet-based compound micro-lenses, Nat. Commun. 8 (2017) 14673.
(26) J. Kim, Joining plasmonics with microfluidics: from convenience to inevitability, Lab Chip. 12 (2012) 3611-3623.
(27) D.R. Griffin, W.M. Weaver, P.O. Scumpia, D. Di Carlo, T. Segura, Accelerated wound healing by injectable microporous gel scaffolds assembled from annealed building blocks, Nat. Mater. 14 (2015) 737-744. doi:10.1038/nmat4294.
(28) E. Sideris, D.R. Griffin, Y. Ding, S. Li, W.M. Weaver, D. Di Carlo, T. Hsiai, T. Segura, Particle Hydrogels Based on Hyaluronic Acid Building Blocks, ACS Biomater. Sci. Eng. 2 (2016) 2034-2041. doi:10.1021/acsbiomaterials.6b00444.
(29) J.E. Mealy, J.J. Chung, H.H. Jeong, D. Issadore, D. Lee, P. Atluri, J.A. Burdick, Injectable Granular Hydrogels with Multifunctional Properties for Biomedical Applications, Adv. Mater. 30 (2018) 1-7. doi:10.1002/adma.201705912.
(30) A. Sheikhi, J. de Rutte, R. Haghniaz, O. Akouissi, A. Sohrabi, D. Di Carlo, A. Khademhosseini, Microfluidic-enabled bottom-up hydrogels from annealable naturally-derived protein microbeads, Biomaterials. 192 (2019) 560-568.
(31) A.G. Floyd, Top ten considerations in the development of parenteral emulsions, Pharm. Sci. Technolo. Today. 2 (1999) 134-143.
(32) S. Ganta, M. Talekar, A. Singh, T.P. Coleman, M.M. Amiji, Nanoemulsions in translational research-opportunities and challenges in targeted cancer therapy, Aaps Pharmscitech. 15 (2014) 694-708.
(33) I.F. Guha, K.K. Varanasi, Separating nanoscale emulsions: Progress and challenges to date, Curr. Opin. Colloid Interface Sci. (2018).
(34) R. Deshmukh, P. Wagh, J. Naik, Solvent evaporation and spray drying technique for micro-and nanospheres/particles preparation: A review, Dry. Technol. 34 (2016) 1758-1772.
(35) A.R. do Vale Morais, É. do Nascimento Alencar, F.H.X. Júnior, C.M. de Oliveira, H.R. Marcelino, G. Barratt, H. Fessi, E.S.T. do Egito, A. Elaissari, Freeze-drying of emulsified systems: A review, Int. J. Pharm. 503 (2016) 102-114.
(36) L. Rey, J.C. May, Freeze-Drying/Lyophilization Of Pharmaceutical & Biological Products, Revised and Expanded, CRC Press, 2004.
(37) J.C. Kasper, G. Winter, W. Friess, Recent advances and further challenges in lyophilization, Eur. J. Pharm. Biopharm. 85 (2013) 162-169.
(38) P. Fonte, S. Reis, B. Sarmento, Facts and evidences on the lyophilization of polymeric nanoparticles for drug delivery, J. Control. Release. 225 (2016) 75-86.
(39) C. Zhang, F. Feng, H. Zhang, Emulsion electrospinning: Fundamentals, food applications and prospects, Trends Food Sci. Technol. (2018).
(40) L.G. Gómez-Mascaraque, A. López-Rubio, Protein-based emulsion electro sprayed micro-and submicroparticles for the encapsulation and stabilization of thermosensitive hydrophobic bioactives, J. Colloid Interface Sci. 465 (2016) 259-270.
(41) M.K. Lee, M.Y. Kim, S. Kim, J. Lee, Cryoprotectants for freeze drying of drug nano-suspensions: effect of freezing rate, J. Pharm. Sci. 98 (2009) 4808-4817.
(42) T. Kawakatsu, Y. Kikuchi, M. Nakajima, Regular-sized cell creation in microchannel emulsification by visual microprocessing method, J. Am. Oil Chem. Soc. 74 (1997) 317-321.
(43) E. Amstad, M. Chemama, M. Eggersdorfer, L.R. Arriaga, M.P. Brenner, D.A. Weitz, Robust scalable high throughput production of monodisperse drops, Lab Chip. 16 (2016) 4163-4172.
(44) S. Sugiura, M. Nakajima, S. Iwamoto, M. Seki, Interfacial tension driven monodispersed droplet formation from microfabricated channel array, Langmuir. 17 (2001) 5562-5566.
(45) J.M. de Rutte, J. Koh, D. Di Carlo, Scalable high-throughput production of modular microgels for in situ assembly of microporous tissue scaffolds, Submitted. (2019).

## Claims

1. A method of making a lyophilized composition, the method comprising:
combining a microfluidic-made lyophilizable agent dispersed within an aqueous phase with an oil and a surfactant so as to form a multiphase system wherein the lyophilizable agent dispersed within the aqueous phase is stabilized within a continuous oil/surfactant phase, wherein the oil is heat conductive and wherein the oil and/or the surfactant exhibits a boiling point at 1 atmosphere that is above 50 °C and/or a pour point that is at least above -50°C;
freezing the dispersed phase of the multiphase system; and
removing the water, oil and surfactant from the multiphase system using a drying process;
so that the lyophilized composition is made.

2. The method of claim 1, wherein the drying process comprises a vacuum mediated drying process and/or a heat mediated drying process.

3. The method of claim 1, wherein the multiphase system is cooled to at least about -78 °C, or at least about -195°C.

4. The method of claim 1, wherein the lyophilizable agent comprises beads, particles, polymer precursors, polymers, polynucleotides or polypeptides.

5. The method of claim 1, wherein the lyophilizable agent comprises hydrogel particles.

6. The method of claim 1, wherein the lyophilizable agent comprises polymeric particles or annealable microbeads.

7. The method of claim 1, wherein the lyophilizable agent comprises gelatin methacryloyl (GelMA) or PEG-vinylsulfone (PEGVS).

8. The method of claim 1, wherein the oil remains as a liquid when the dispersed phase is frozen.

9. The method of claim 1, wherein the oil is also frozen when the dispersed phase is frozen.

10. The method of claim 1, further comprising resuspending the lyophilized composition in an aqueous resuspension solution.

11. The method of claim 10, wherein the aqueous resuspension solution comprises a crosslinking agent.

12. A method of making a resuspended lyophilized composition comprising:
making a lyophilized composition using the steps of any one of claims 1 to 9 and combining the lyophilized composition with an aqueous resuspension solution such that a resuspended lyophilized composition is made.

13. The method of claim 12, wherein the lyophilizable agent comprises hydrogel particles.

14. The method of claim 13, wherein the aqueous resuspension solution comprises a crosslinking agent.

15. The method of claim 14, further comprising crosslinking the hydrogel particles in the resuspended lyophilized composition so as to form a microporous hydrogel scaffold.

## Patentansprüche

1. Ein Verfahren zur Herstellung einer lyophilisierten Zusammensetzung, das Verfahren beinhaltend:
Kombinieren eines mikrofluidisch-hergestellten lyophilisierbaren Wirkstoffs, der in einer wässrigen Phase dispergiert ist, mit einem Öl und einem Tensid, sodass ein Multiphasen-System gebildet wird, wobei der lyophilisierbare Wirkstoff, der in der wässrigen Phase dispergiert ist, in einer kontinuierlichen Öl-/Tensidphase stabilisiert ist, wobei das Öl wärmeleitend ist und wobei das Öl und/oder das Tensid einen Siedepunkt bei 1 Atmosphäre aufweist, der über 50 °C liegt, und/oder einen Stockpunkt, der mindestens über -50 °C liegt;
Gefrieren der dispergierten Phase des Multiphasen-Systems; und
Entfernen des Wassers, Öls und Tensids aus dem Multiphasen-System unter Verwendung eines Trocknungsprozesses;
so dass die lyophilisierte Zusammensetzung hergestellt wird.

2. Verfahren gemäß Anspruch 1, wobei der Trocknungsprozess einen vakuumvermittelten Trocknungsprozess und/oder einen wärmevermittelten Trocknungsprozess beinhaltet.

3. Verfahren gemäß Anspruch 1, wobei das Multiphasen-System auf mindestens etwa -78 °C oder mindestens etwa -195 °C gekühlt wird.

4. Verfahren gemäß Anspruch 1, wobei der lyophilisierbare Wirkstoff Kügelchen, Partikel, Polymervorläufer, Polymere, Polynucleotide oder Polypeptide beinhaltet.

5. Verfahren gemäß Anspruch 1, wobei der lyophilisierbare Wirkstoff Hydrogel-Partikel beinhaltet.

6. Verfahren gemäß Anspruch 1, wobei der lyophilisierbare Wirkstoff Polymerpartikel oder vergütbare Mikrokügelchen beinhaltet.

7. Verfahren gemäß Anspruch 1, wobei der lyophilisierbare Wirkstoff Gelatine-Methacryloyl (GelMA) oder PEG-Vinylsulfon (PEGVS) beinhaltet.

8. Verfahren gemäß Anspruch 1, wobei das Öl flüssig bleibt, wenn die dispergierte Phase gefroren ist.

9. Verfahren gemäß Anspruch 1, wobei das Öl auch gefroren ist, wenn die dispergierte Phase gefroren ist.

10. Verfahren gemäß Anspruch 1, ferner beinhaltend Resuspendieren der lyophilisierten Zusammensetzung in einer wässrigen Resuspensionslösung.

11. Verfahren gemäß Anspruch 10, wobei die wässrige Resuspensionslösung ein Vernetzungsmittel beinhaltet.

12. Ein Verfahren zur Herstellung einer resuspendierten lyophilisierten Zusammensetzung, beinhaltend:
Herstellen einer lyophilisierten Zusammensetzung unter Verwendung der Schritte gemäß einem der Ansprüche 1 bis 9 und Kombinieren der lyophilisierten Zusammensetzung mit einer wässrigen Resuspensionslösung, so dass eine resuspendierte lyophilisierte Zusammensetzung hergestellt wird.

13. Verfahren gemäß Anspruch 12, wobei der lyophilisierbare Wirkstoff Hydrogel-Partikel beinhaltet.

14. Verfahren gemäß Anspruch 13, wobei die wässrige Resuspensionslösung ein Vernetzungsmittel beinhaltet.

15. Verfahren gemäß Anspruch 14, ferner beinhaltend Vernetzen der Hydrogel-Partikel in der resuspendierten lyophilisierten Zusammensetzung, so dass ein mikroporöses Hydrogel-Gerüst gebildet wird.

## Revendications

1. Un procédé de réalisation d'une composition lyophilisée, le procédé comprenant :
la combinaison d'un agent lyophilisable réalisé de manière microfluidique dispersé dans une phase aqueuse avec une huile et un tensioactif de façon à former un système multiphase dans lequel l'agent lyophilisable dispersé dans la phase aqueuse est stabilisé dans une phase huile/tensioactif continue, dans lequel l'huile est thermoconductrice et dans lequel l'huile et/ou le tensioactif présente(nt) un point d'ébullition à 1 atmosphère qui est supérieur à 50 °C et/ou un point d'écoulement qui est au moins supérieur à -50 °C ;
le gel de la phase dispersée du système multiphase ; et
l'élimination de l'eau, de l'huile et du tensioactif du système multiphase à l'aide d'un processus de séchage ;
de sorte à réaliser la composition lyophilisée.

2. Le procédé de la revendication 1, dans lequel le processus de séchage comprend un processus de séchage médié par le vide et/ou un processus de séchage médié par la chaleur.

3. Le procédé de la revendication 1, dans lequel le système multiphase est refroidi jusqu'à au moins environ -78 °C, ou au moins environ -195 °C.

4. Le procédé de la revendication 1, dans lequel l'agent lyophilisable comprend des billes, des particules, des précurseurs de polymère, des polymères, des polynucléotides ou des polypeptides.

5. Le procédé de la revendication 1, dans lequel l'agent lyophilisable comprend des particules d'hydrogel.

6. Le procédé de la revendication 1, dans lequel l'agent lyophilisable comprend des particules de polymère ou des microbilles pouvant être recuites.

7. Le procédé de la revendication 1, dans lequel l'agent lyophilisable comprend du méthacryloyle de gélatine (GelMA) ou de la PEG-vinylsulfone (PEGVS).

8. Le procédé de la revendication 1, dans lequel l'huile reste liquide lorsque la phase dispersée est gelée.

9. Le procédé de la revendication 1, dans lequel l'huile est également gelée lorsque la phase dispersée est gelée.

10. Le procédé de la revendication 1, comprenant en outre la re-suspension de la composition lyophilisée dans une solution de re-suspension aqueuse.

11. Le procédé de la revendication 10, dans lequel la solution de re-suspension aqueuse comprend un agent de réticulation.

12. Un procédé de réalisation d'une composition lyophilisée re-suspendue comprenant : la réalisation d'une composition lyophilisée en utilisant les étapes de l'une quelconque des revendications 1 à 9 et en combinant la composition lyophilisée avec une solution de re-suspension aqueuse de telle sorte à réaliser une composition lyophilisée re-suspendue.

13. Le procédé de la revendication 12, dans lequel l'agent lyophilisable comprend des particules d'hydrogel.

14. Le procédé de la revendication 13, dans lequel la solution de re-suspension aqueuse comprend un agent de réticulation.

15. Le procédé de la revendication 14, comprenant en outre la réticulation des particules d'hydrogel dans la composition lyophilisée re-suspendue de façon à former un échafaudage d'hydrogel microporeux.
